(19) Europäisches Patentamt
European Patent Office
Office européen des brevets

(11) Publication number: 0 096 330
A2

# (12) EUROPEAN PATENT APPLICATION

(21) Application number: 83105335.0

(22) Date of filing: 30.05.83

(51) Int. Cl.$^3$: A 61 B 5/04
H 01 B 1/12

(30) Priority: 31.05.82 JP 91469/82

(43) Date of publication of application:
21.12.83 Bulletin 83/51

(84) Designated Contracting States:
DE FR GB IT

(71) Applicant: TORAY INDUSTRIES, INC.
2, Nihonbashi-Muromachi 2-chome Chuo-ku
Tokyo 103(JP)

(72) Inventor: Kenjo, Hideki
4-19-3, Sonoyama 2-chome
Otsu-shi Shiga-ken(JP)

(72) Inventor: Komaki, Kazushige
125-18, Setahashimoto-cho
Otsu-shi Shiga-ken(JP)

(74) Representative: Kador . Klunker . Schmitt-Nilson . Hirsch
Corneliusstrasse 15
D-8000 München 5(DE)

## (54) Electrode for measuring electroretinogram.

(57) An electrode for measuring electroretinograms which is made of a lens-shaped material composed of an electroconductive hydrogel and a lead line connected to the lens-shaped material in the portion thereof not falling in contact with an eyeball.

EP 0 096 330 A2

Croydon Printing Company Ltd

# ELECTRODE FOR MEASURING ELECTRORETINOGRAM

## BACKGROUND OF THE INVENTION

### (1) Field of the Invention

This invention relates to a lens-shaped electrode for measuring a electroretinogram (hereinafter referred to as "ERG" for brevity).

### (2) Description of the Prior Art

Electroretinography is a means for measuring the potential of the retina for inspecting whether or not the function of the retina is normal. This means utilizes the phenomenon of the electric response of the retina to light stimulus and is indispensable for clinical examinations in ophthalmology.

For example, in the case of cataracts, if the function of the retina is normal, acuteness of vision can be restored by removing the opaque crystalline lens and appropriately adjusting the visual power. Accordingly, for a cataract operation, it is necessary to check before the operation whether or not the function of the retina is normal. However, direct observation of the retina of a patient suffering from a cataract is impossible because the crystalline lens of the patient is opaque. Therefore, EGR is utilized for this purpose.

Since the potential of the retina is transmitted to the cornea and the bulbar conjunctiva, the potential of the retina need not directly be measured. It is sufficient if the potential of the cornea or bulbar conjunctiva is measured. As the ERG-measuring electrode for inspecting variations of the potential from these portions, there are known a corneal electrode and a noncorneal electrode. As the electrode of the former type, there are known an electrode using a contact lens and an electrode not using a contact lens. Two methods are practically adopted for measuring ERG using these electrodes.

In the first method, a hard contact lens

(hereinafter referred to as "HCL" for brevity) is used. A foil or line of gold or platinum is attached to the surface of the hard contact lens so as to contact the cornea. The potential is measured through a lead line piercing through the HCL.

In the second method, an electrode is directly inserted between the lower eyelid and the bulbar conjunctiva.

The former HCL method is advantageous in that variations of the potential can easily be inspected, but is defective in that the lens is uncomfortable to the patient and readily damages the surface of the cornea. The latter electrode-inserting method is advantageous in that the lens does not damage the surface of the cornea, but is defective in that the variation of the potential is too small to precisely inspect and the inserted electrode is also very uncomfortable to the patient.

SUMMARY OF THE INVENTION

It is therefore a primary object of the present invention to provide an electrode for measuring ERG, by which variations of the potential can easily be inspected which is not uncomfortable to the patient, and which does not damage the surface of the cornea.

In accordance with the present invention, there is provided an electrode for measuring ERG, which comprises a lens-shaped material composed of an electroconductive hydrogel and a lead line connected to the lens shaped material in the portion thereof not falling in contact with an eyeball.

DESCRIPTION OF THE PREFERRED EMBODIMENTS

The electrode for measuring ERG according to the present invention comprises a lens-shaped material composed of an electroconductive hydrogel and a lead line connected to the substance in the portion not falling in contact with an eyeball.

The electroconductive hydrogel used in the present

invention has an electric resistance not more than 1 mega-Ω·cm, preferably not more than 1 kilo-Ω·cm, and a water content of 30% to 95% by weight, preferably 50% to 90% by weight. If the electric resistance exceeds 1 mega-Ω·cm, the variation of the potential, which is inherently small, is further reduced. Thus, it becomes difficult or even impossible to detect the variation of the potential. If the water content is lower than 30% by weight, the electric resistance is ordinarily increased and the object of the present invention cannot be attained.

The hydrogel is formed of a polymeric material which has a light-transmitting property and also has an intermolecular bond sufficient to retain the shape. The polymeric material is preferably hydrophilic.

Natural polymers and synthetic polymers can be used as the hydrophilic polymer. Agar-agar and devil's-tongue can be used as the natural polymer. Ordinarily, synthetic polymers are preferable to natural polymers in reproducibility, degree of purification, and easiness in heat sterilization after the molding. Any of synthetic hydrophilic polymers customarily used for the production of soft contact lenses can be used in the present invention. For example, there can be mentioned homopolymers of vinyl lactams such as N-vinyl-pyrrolidone; acrylic compounds such as acrylamide, dimethylacrylamide, diethylacrylamide, N-propyloxymethylacrylamide, and dimethylmethacrylamide; vinylamines such as vinylpyridine; vinyl compounds such as vinyl-sulfonic acid and styrene-sulfonic acid; and compounds represented by the following formula:

$$R_1-(CH_2-CH_2-O)_n-\underset{\underset{O}{\|}}{C}-\underset{\underset{R_2}{|}}{C}=CH_2$$

wherein $R_1$ is -OH, $-OCH_3$ or $-O-\underset{\underset{O}{\|}}{C}-\underset{\underset{R_2}{|}}{C}=CH_2$ , $R_2$ is -H or $-CH_3$ ,

and n is 0 or an integer of at least 1; and copolymers of two or more of the foregoing monomers. The compounds of the above formula can be used as is in an unpolymerized form as the hydrophilic high molecular weight compound, if the value of n is sufficiently large.

It is known that the mechanical strength of hydrogels is ordinarily reduced with an increase of the water content. In order to obtain a gel having an increased mechanical strength at a high water content, it is preferred that the hydrophilic polymer be blended with a hydrophobic polymer or that a copolymer comprising hydrophilic segments and hydrophobic segments be used.

Homopolymers or copolymers such as mentioned above can be used as the hydrophilic polymer for the formation of such blends. Polymers derived from at least one monomer selected from methacrylic acid esters, acrylic acid esters, methacrylonitrile, acrylonitrile, and aromatic olefins such as styrene are used as the hydrophobic polymer for the formation of such blends. Copolymers comprising hydrophilic segments and hydrophobic segments can be obtained by copolymerization of hydrophilic polymer-forming monomers such as mentioned above with hydrophobic polymer-forming monomers such as mentioned above.

The blend ratio of the hydrophilic polymer to the hydrophobic polymer or the copolymerization ratio of the hydrophilic segments to the hydrophobic segments is ordinarily in the range of from 95/5 to 40/60 by weight.

If the proportion of the hydrophilic polymer or hydrophilic segments exceeds the above range, a lens-shaped material having high mechanical strengths is difficult to prepare. If the proportion of the hydrophilic polymer or hydrophilic segments is below the above-mentioned range, a lens-shaped material having a high water content is difficult to prepare.

As pointed out hereinbefore, it is ordinarily

preferable that the polymer constituting the hydrogel of the present invention be hydrophilic. However, even a hydrophobic polymer can be used if it has a water--containing property. For example, a gel obtained by mixing isotactic polymethyl methacrylate with syndiotactic or atactic polymethyl methacrylate can be used.

The polymer constituting the gel should have an intermolecular bond. The intermolecular bond gives a shape-retaining property to the gel, prevents dissolution of the gel in water, and improves the light-transmitting property of the gel.

The intermolecular bond can be formed, for example, by the following methods.

(1) A cross-linking agent is incorporated in the polymerization system.

As the cross-linking agent, there are used compounds containing in the molecule at least two functional groups readily copolymerizable with the monomers used. For example, there can be mentioned compounds represented by the following formula:

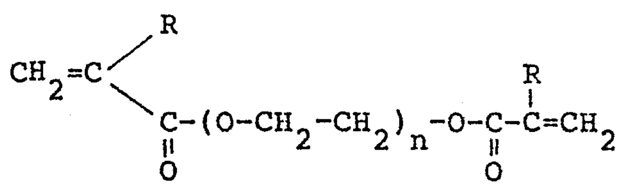

wherein R is -H or $-CH_3$ and n is an integer of at least 1. There can also be mentioned di- or tri-allyl compounds such as diallyl succinate, diallyl phthalate, diallyl maleate, diethylene glycol bisallylcarbonate, triallyl cyanurate, triallyl isocyanurate, triallyl phosphate, and triallyl trimellitate; di- or tri-vinyl compounds such as divinylbenzene, N,N'--methylene-bis-acrylamide, divinylurea, bisphenol A bismethacrylate, divinyl adipate, glycerin trimethacrylate, trimethylolpropane trimethacrylate, trimethylolpropane triacrylate, and trivinyl trimellitate; allylvinyl compounds such as allyl

acrylate and allyl methacrylate; and vinyl methacrylate and vinyl acrylate. Furthermore, in case a hydrogel--forming material comprising a polymer is used, cross-linking can be accomplished by introducing a post-cross-linkable functional group such as a vinyl group, a methacrylic group, or an acrylic group into the polymer.

(2) An ionic group is introduced into a monomer or polymer to form an ionic bond or hydrogen bond.

For example, an ionic complex of vinylpyrrolidone with methacrylic acid or acrylic acid and an ionic bond of an acidic group with a polyvalent metal ion having a valency of at least 2 can be mentioned.

As a preferred material for the ERG measuring electrode, there can be mentioned a blend of poly-N--vinylpyrrolidone as the hydrophilic component with a hydrophobic component selected from a polymer of an alkyl methacrylate having 1 to 4 carbon atoms in the alkyl group, preferably poly(methyl methacrylate), a polymer of an alkyl acrylate having 1 to 4 carbon atoms in the alkyl group, preferably poly(methyl acrylate), and a copolymer of an alkyl acrylate having 1 to 4 carbon atoms in the alkyl group, preferably methyl methacrylate, with an alkyl acrylate having 1 to 4 carbon atoms in the alkyl group, preferably methyl acrylate. Furthermore, a copolymer of N-vinylpyrrolidone with an alkyl methacrylate having 1 to 4 carbon atoms in the alkyl group, preferably methyl methacrylate, and/or an alkyl acrylate having 1 to 4 carbon atoms in the alkyl group preferably methyl acrylate, is preferably used as the material for the ERG measuring electrode. These polymeric materials are cross-linked preferably according to the above-mentioned method (1).

The shape of the material is not particularly critical, so far as it is lens-shaped so that it can be

attached between the eyeball and the eyelid. The power of the lens-shaped material may be zero.

The lead line used for detecting variation of the potential is preferably composed of a material which is excellent in electrical conductivity and does not undergo decomposition or corrosion in a physiological saline solution, such as carbon fiber, gold, platinum, silver, or stainless steel. In case of a lead line composed of a metal, if a light stimulus is strong, a photoelectric effect is undesirably produced. Accordingly, in this respect, carbon fiber is advantageously used. In view of the operation adaptability, it is preferable that the lead line be covered with an non-conductive material.

The following two methods can be mentioned for the production of the lens-shaped electrode for measuring ERG.

(1) A hydrogel-forming material is shaped into a lens-shaped material and thereafter the lead line is attached thereto. This preliminary shaping is accomplished by (a) the method customarily adopted for preparing soft contact lenses, that is, the method comprising cutting a hydrogel-forming polymer bulk into a predetermined lens shape, preferably followed by polishing, (b) the spin-casting method in which a hydrogel-forming polymerizable material is cast in a concave mold while it is maintained in a predetermined shape by rotating the mold, or (c) the method in which a hydrogel-forming polymerizable material is cast between convex and concave molds. The lead line is attached to the so-prepared lens-shaped material, for example, by the method in which the lead line is bonded to the lens-shaped material with an adhesive before inclusion of water, the method in which the lead line is bonded to the lens-shaped material with an adhesive after inclusion of water, or the method in which the lead line is stabbed into the lens-shaped material

after inclusion of water. However, these lead line--attaching methods are not preferable in that the hydrogel is readily deformed or the lead line is likely to come off.

(2) A hydrogen-forming material is cast or molded into a lens shape, integrally with the lead line between convex and concave molds. According to this method, the lens-shaped material having the same shape as that of the final electrode can be formed integrally with the lead line. This method is suitable for mass production. Furthermore, a concave mold with a groove or the like formed in the concave surface is preferably used. This is because it enables to connect the lead line exactly to the intended portion of the lens-shaped material, not falling in contact with an eyeball, by placing the lead line in the groove, whereby damage of the cornea with the lead line can be avoided. The lens-shaped electrode prepared by this method is most suitable for the clinical treatment. Therefore, the adoption of this integral molding method is preferable.

The present invention will now be described in detail with reference to the following examples that by no means limit the scope of the invention. In the examples percents are by weight unless otherwise specified.

Example 1

Molds

A glass ball was used as the convex mold, and a concave mold composed of polypropylene was used and a groove was formed so that a carbon fiber lead line was extended to the outside.

Lead Line

Twenty carbon fibers (each 10 μm in diameter and 5 cm in length) were gathered together and were bonded together along the central portion of 3 cm by using a silicone type adhesive.

Starting Polymerizable Material Solution

| | |
|---|---|
| Methyl methacrylate | 30 parts |
| N-vinylpyrrolidone | 75 parts |
| Triallyl isocyanurate | 1.0 part |
| Triethylene glycol dimethacrylate | 3.0 parts |
| Azobisisobutyronitrile | 0.1 part |
| Dimethyl sulfoxide | 400 parts |

The lead line was placed in the concave mold so that one end of the lead line was located at the center of the concave mold. Then, the starting polymerizable material solution was cast in the concave mold and the convex mold was placed thereon. In an argon atmosphere, the assembly mold was placed in a glass flask and the flask was covered with a lid. The temperature was elevated to effect the polymerization. After the polymerization, the formed gel was taken out in an aqueous solution of dimethyl sulfoxide. After the substitution with water, the water content of the gel was measured. It was found that the water content of the gel was 73%.

When this electrode was compared with an electrode composed of a hard contact lens with respect to the measurement of ERG, it was found that both the electrodes had the same capacities except that signals due to the photoelectric effect appeared in case of the hard contact lens electrode. When the surface of the cornea was examined by a slit lamp after the measurement of ERG, it was found that the surface of the cornea was not hurt at all in case of the electrode of this example.

Example 2

Molds

A stainless steel ball was used as the convex mold, and the same concave mold as used in Example 1 was used.

Lead Line

A gold line having a diameter of 30 μm and a

length of 3 cm was used.

Starting Polymerizable Material Solution

| | |
|---|---|
| Polymethyl methacrylate containing 1% of a vinyl group as the post-crosslinkable group | 35 parts |
| N-vinylpyrrolidone | 50 parts |
| Triallyl isocyanurate | 0.25 part |
| Azobisdimethylvaleronitrile | 0.1 part |
| Dimethyl sulfoxide | 158 parts |

The gold line was inserted into the concave mold. The polymerization was carried out in the same manner as described in Example 1 to obtain an ERG measuring electrode having a water content of 65%. When this electrode was used for the measurement of ERG, the same capacity as obtained in case of the hard contact lens electrode was obtained.

<u>Example 3</u>

Molds

Convex and concave molds composed of glass were used.

Lead Line

The same carbon fiber lead line as used in Example 1 was used.

Starting Polymerizable Material Solution

| | |
|---|---|
| Methyl acrylate | 20 parts |
| Methyl methacrylate | 30 parts |
| Methoxypolyethylene glycol monomethacrylate of the following formula: | 50 parts |

$$CH_2=\overset{CH_3}{\overset{|}{C}}-\underset{O}{\underset{\|}{C}}O(CH_2-CH_2-O)_{23}-CH_3$$

| | |
|---|---|
| Triethylene glycol dimethacrylate | 0.5 part |
| Azobisdimethylvaleronitrile | 0.1 part |
| Dimethyl sulfoxide | 360 parts |

The cast polymerization was carried out in the same manner as described in Example 1 to obtain an electrode having a water content of 76%, which was excellent in the light-transmitting property and had

the same shape as defined by the molds.

Example 4

The cast polymerization was carried out in the same manner as described in Example 1 except that a starting polymerizable material solution having the following composition was used:

| | |
|---|---|
| Polymethyl methacrylate containing a vinyl group as the post-cross-linking functional group | 32 parts |
| N-Vinylpyrrolidone | 300 parts |
| Triallyl isocyanurate | 1 part |
| Azobisdimethylvaleronitrile | 0.1 part |
| Dimethyl sulfoxide | 1177 parts |

The obtained electrode had a water content of 93% by weight and was very soft and excellent in transparency.

Example 5

The cast polymerization was carried out in the same manner as described in Example 1 except that a starting polymerizable material solution having the following composition was used:

| | |
|---|---|
| Methoxypolyethylene glycol monomethacrylate of the following formula: | 100 parts |

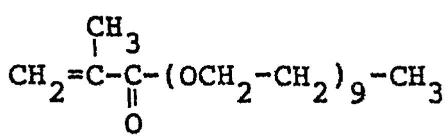

| | |
|---|---|
| Ethylene glycol dimethacrylate | 0.3 part |
| Azobisisobutyronitrile | 0.1 part |
| Dimethyl sulfoxide | 170 parts |

The obtained electrode had a water content of 84% and was excellent in the transparency as the electrode of Example 4.

CLAIMS

1. An electrode for measuring electroretinograms, which comprises a lens-shaped material composed of an electroconductive hydrogel and a lead line connected to the lens-shaped material in the portion thereof not falling in contact with an eyeball.

2. An electrode as set forth in claim 1, wherein the electric resistance of the electroconductive hydrogel is not more than 1 mega-Ω·cm.

3. An electrode as set forth in claim 1, wherein the electric resistance of the electroconductive hydrogel is not more than 1 kilo-Ω·cm.

4. An electrode as set forth in claim 1, wherein the water content of the electroconductive hydrogel is in the range of from 30 to 95% by weight.

5. An electrode as set forth in claim 1, wherein the hydrogel is composed of a mixture of poly-N--vinylpyrrolidone with a polymer of an alkyl methacrylate having 1 to 4 carbon atoms in the alkyl group, a polymer of an alkyl acrylate having 1 to 4 carbon atoms in the alkyl group or a copolymer of an alkyl methacrylate having 1 to 4 carbon atoms in the alkyl group with an alkyl acrylate having 1 to 4 carbon atoms in the alkyl group.

6. An electrode as set forth in claim 1, wherein the hydrogel is composed of a copolymer of N-vinylpyrrolidone with at least one monomer selected from the group consisting of an alkyl methacrylate having 1 to 4 carbon atoms in the alkyl group and an alkyl acrylate having 1 to 4 carbon atoms in the alkyl group.

7. An electrode as set forth in claim 1, wherein the lead line is made of carbon fiber, platinum, gold, silver or stainless steel.

8. An electrode as set forth in claim 1, wherein said electrode is prepared by casting or molding a hydrogel-forming material into a lens-shaped material,

integrally with a lead line between convex and concave molds.

9. An electrode as set forth in claim 1, wherein the concave mold has a groove.

10. A method of preparing an electrode of claim 1, which comprises casting or molding a hydrogel-forming material into a lens-shaped material, integrally with a lead line between convex and concave molds.

11. A method as set forth in claim 8, wherein the concave mold has a groove.